# FASCICULE DE BREVET EUROPEEN

(11) **EP 4 277 672 B1**
(45) Date de publication et mention de la délivrance du brevet: **18.06.2025**
(21) Numéro de dépôt: 22703022.8
(22) Date de dépôt: 13.01.2022
(51) Int. Cl.: A61L 27/06, A61L 27/30, A61L 27/34, A61L 27/54

(54) **IMPLANT ET SON PROCEDE DE FABRICATION**
IMPLANTAT UND VERFAHREN ZU DESSEN HERSTELLUNG
IMPLANT AND METHOD FOR PRODUCING SAME

(30) Priorité: 13.01.2021 FR 2100312
(43) Date de publication de la demande: 22.11.2023
(73) Titulaire: Activ' Biomat, 95957 Roissy Aeroport CDG (FR)
(72) Inventeur: BLANQUAERT, Daniel, 75008 PARIS (FR); LEROUX, Amélie, 95120 ERMONT (FR); MIGONNEY, Véronique, 95600 EAUBONNE (FR)
(74) Mandataire: CAPRI
(86) Numéro de dépôt international: PCT/FR2022/050071
(87) Numéro de publication internationale: WO 2022/153012

(56) Documents cités:
- FR-A1- 2 789 315
- FR-A1- 3 042 715
- US-A1- 2005 048 193
- MAYINGI J ET AL: "Synthese et greffage de polymeres bioactifs sur des surfaces en titane pour favoriser l'osteointegration", IRBM, ELSEVIER, AMSTERDAM, NL, vol. 29, no. 1, 1 March 2008 (2008-03-01), pages 1 - 6, XP022832498, ISSN: 1959-0318, [retrieved on 20080304], DOI: 10.1016/J.RBMRET.2007.10.001

## Description

La présente invention concerne un implant et un procédé de fabrication de cet implant. L'implant de l'invention est destiné à venir en contact direct des tissus osseux, sans utilisation de ciment, résine, colle, etc. L'objectif est que les tissus osseux se développent et repoussent à la surface et en profondeur de l'implant par ostéointégration. Par implant, il faut comprendre toute pièce, ensemble de pièces ou dispositifs destinés à être implantés, partiellement ou en totalité, dans un corps humain ou animal. On peut par exemple citer les implants dentaires, les prothèses de hanche, les prothèses de genou, les prothèses d'épaule, les cages intervertébrales, etc.

Dans les implants fémoraux par exemple, on connait les cotyles en titane ou en alliage de titane destinés à être implantés dans une cavité de l'os iliaque. Le titane ou l'alliage de titane constitutif des cotyles est très dense : il n'est pas poreux. Ces cotyles présentent en général une forme externe hémisphérique destinée à venir en contact de la cavité osseuse. La surface externe hémisphérique du cotyle peut être structurée, par exemple avec un réseau quadrillé de rainures, délimitant ainsi des petits pavés saillants. Cette macrostructure augmente la surface spécifique de contact à l'os, ce qui renforce la stabilité du cotyle dans la cavité osseuse, mais n'est pas suffisante pour permettre une repousse osseuse. Il est fréquent de revêtir la surface externe du cotyle (structurée ou non) avec de l'hydroxyapatite, composant minéral naturellement présent dans l'os, afin d'augmenter son ostéointégration. Toutefois, ce revêtement d'hydroxyapatite ne permet pas une réponse contrôlée de l'hôte et une réaction à « corps étranger » peut intervenir.

D'autre part, il est connu du document EP2032663 de greffer du PolyNaSS (polystyrène sulfonate de sodium) sur des implants en titane ou en alliage de titane, dans le but de favoriser la biointégration des implants dans les tissus en permettant une régénération osseuse, tout en prévenant des infections, grâce à ses caractéristiques chimiques de mime des glycosaminoglycanes, qui évitent l'adhésion des bactéries et la création du biofilm bactérien.

Il n'est pas possible de greffer du PolyNaSS sur un cotyle revêtu d'hydroxyapatite. En revanche, il est possible de greffer du PolyNaSS sur un cotyle dépourvu d'hydroxyapatite.

La présente invention a pour but de remédier aux inconvénients précités en définissant un implant en titane ou en alliage présentant une rugosité optimale, de manière à pouvoir être revêtu de PolyNaSS par greffage, afin de permettre une repousse osseuse contrôlée et éviter le biofilm bactérien et l'infection.

Dans l'art antérieur, on connait le document FR2789315A1 de 1999 qui décrit un implant en alliage de titane qui est revêtu :
- d'une couche poreuse interne présentant une épaisseur de 60 à 100 µm, une porosité inférieure à 10 % et une rugosité inférieure à 50 µm, et
- d'une couche poreuse externe présentant une épaisseur de 60 à 100 µm, une porosité de 10 à 20 % et une rugosité de 100 à 150 µm.

L'implant ainsi formé est ensuite revêtu d'hydroxyapatite de calcium.

Dans l'art antérieur, on connait également la publication de 2008 intitulée Mayingi et al : « Synthèse et greffage de polymères bioactifs sur des surfaces en titane pour favoriser l'ostéointégration », IRBM, ELSEVIER, AMSTERDAM NL, vol.29, n °1, 1 mars 2008 (2008-03-01), pages 1-6, XP022832498, ISSN : 1959-0318, DOI: 10.1016/J.RBMRET.2007.10.001 (extrait le 2008-03-04*).* Dans cette publication de 2008, il est prévu de greffer du PolyNaSS sur du titane ou un alliage de titane. Les caractéristiques du titane (ou alliage de titane) ne sont pas précisées dans cette publication.

Le document FR2902102A1 de 2006 traite aussi du greffage de PolyNaSS sur du titane. Le rapport de recherche de ce document FR2902102A1 cite un document EP0893164A2 de 1998 qui traite déjà du greffage de NaSS.

La présente invention propose un implant comprenant un corps dense en titane ou alliage de titane, qui est revêtu d'une couche poreuse en titane ou alliage de titane, similaire à celle du document FR2789315A1 de 1999, mais sur laquelle on a déposé du PolyNaSS par greffage, comme dans le document EP0893164A2 de 1998, à la place de l'hydroxyapatite de calcium.

On peut ainsi remarquer que plus de 20 années se sont écoulées entre la publication des documents FR2789315A1 et EP0893164A2 et le dépôt de présente demande. Et si l'on considère le document FR2902102A1 de 2006, 15 années se sont quand même écoulées. Cela implique que le greffage de PolyNaSS sur une couche poreuse de titane n'est pas évident du tout pour les spécialistes du domaine des implants. Autrement, cette technologie aurait été décrite et mise œuvre depuis longtemps. Il faut voir dans cette longue période un indice flagrant d'activité inventive.

Cette longue période s'explique également par des considérations techniques, et notamment une optimisation de l'oxydation du fait de la porosité de la couche, ce qui permet d'obtenir des taux de greffage 10 à 20 fois plus élevés, facteur essentiel à la régénération osseuse. De plus, on obtient ainsi un matériau hybride composé du titane et d'un tissu vivant l'os, parfaitement intégré grâce à la présence du polyNaSS, qui va en même temps prévenir toute adhérence bactérienne et donc l'infection. Ce véritable matériau composite présente un module d'élasticité progressif entre le titane dense et l'os cortical ou spongieux. Cette structure n'est pas évidente, y compris pour les spécialistes du domaine.

Avantageusement, le corps dense peut présenter une rugosité Ra de 4 µm à 8 µm. En effet, la rugosité du corps dense, qui n'est pas poreux, permet une meilleure accroche de la couche poreuse.

Avantageusement, la couche poreuse peut présenter une épaisseur de l'ordre de 200 µm à 400 µm, avantageusement de l'ordre de 300 µm à 400 µm.

Selon un mode de réalisation abouti, la couche poreuse peut comprendre une couche interne poreuse en contact du corps dense et une couche externe poreuse en contact de la couche interne poreuse, les deux couches poreuses interne et externe présentant des porosités différentes, la couche externe poreuse présentant avantageusement une porosité supérieure à celle de la couche interne poreuse.

La couche interne avec des particules de faibles dimensions permet d'améliorer l'adhésion au corps dense et la couche externe avec des particules de dimensions plus importante permet d'obtenir des diamètres de pores et donc une porosité en volume élevée, facteurs essentiels à une vascularisation sanguine tout azimut et par voie de conséquence une colonisation osseuse optimale.

De préférence, la couche externe poreuse peut présenter une porosité de l'ordre de 10 % à 25 %, avantageusement de l'ordre de 20 %. D'autre part, la couche externe poreuse peut présenter une épaisseur de l'ordre de 150 µm à 300 µm. Par ailleurs, la couche externe poreuse peut être constituée de poudre de titane ou d'alliage de titane ayant une granulométrie comprise entre 90 µm et 250 µm. La couche externe poreuse présente de préférence une rugosité moyenne Ra de l'ordre de 30 à 70 µm et une rugosité maximale Rz de l'ordre de 150 à 300 µm.

La couche externe poreuse est la couche d'ostéo-intégration optimale grâce à sa rugosité et sa porosité. Elle constitue également une couche d'imprégnation optimale pour le PolyNaSS.

De son côté, la couche interne poreuse peut présenter une épaisseur de l'ordre de 50 µm à 100 µm. Elle peut être constituée de poudre de titane ou d'alliage de titane ayant une granulométrie comprise entre 40 µm et 100 µm. La couche interne poreuse peut présenter une adhérence supérieure ou égale à 22 MPa. La couche interne poreuse peut être qualifiée de couche d'adhérence optimale permettant aussi bien une bonne accroche sur le corps dense qu'une bonne accroche de la couche externe poreuse. Il est à noter que la couche interne poreuse peut être déposée sur la surface externe d'un cotyle doté d'une macrostructure, comme exposé ci-dessus.

La présente invention définit également un procédé de fabrication d'un implant comprenant un corps dense en titane ou alliage de titane, caractérisé en ce qu'une couche poreuse de titane ou d'alliage de titane est déposée à la torche à plasma sur le corps dense, du PolyNaSS étant ensuite déposé par greffage sur la couche poreuse.

De préférence, le procédé de fabrication comprend les étapes suivantes :
a- déposer à la torche à plasma de la poudre de titane ayant une granulométrie moyenne de l'ordre de 40 à 100 µm, de manière à former sur le corps dense une couche interne poreuse ayant une épaisseur de l'ordre de 50 µm à 100 µm,
b- déposer à la torche à plasma de la poudre de titane ayant une granulométrie moyenne de l'ordre de 90 à 250 µm, de manière à former sur la couche interne poreuse une couche externe poreuse ayant une épaisseur de l'ordre de 150 µm à 300 µm, et avantageusement une porosité de 10 à 25 %,
c- greffer du PolyNaSS sur la couche externe poreuse.

La torche à plasma permet de déposer les grains de poudre de titane à une température suffisamment élevée pour que leur surface externe soit au moins partiellement en fusion, de sorte que les grains de poudre vont se coller les uns aux autres en laissant des cavités entre eux, d'où la porosité recherchée. Le PolyNaSS est ensuite déposé sur la surface externe accidentée de la couche poreuse externe en pénétrant plus ou moins dans les cavités. On peut considérer que la surface spécifique est au moins multipliée par 2 ou 3, en comparaison avec un cotyle classique macrostructuré revêtu d'hydroxyapatite. Ainsi, une quantité importante de PolyNaSS peut être déposée avec une accroche de qualité.

L'esprit de la présente invention réside dans le fait de créer une surface externe en titane rugueuse, afin de pouvoir y appliquer du PolyNaSS, qui va parfaitement adhérer à cette surface externe en titane et qui va pouvoir remplir pleinement sa fonction de repousse osseuse par ostéointégration et sa fonction d'anti-adhésion bactérienne.

L'invention sera maintenant plus amplement décrite en référence aux dessins joints donnant, à titre d'exemple non limitatif, un mode de réalisation de l'invention.

Sur les figures :
La figure 1 est une vue en perspective légèrement de dessus d'un implant sous la forme d'un cotyle classique en titane ou alliage de titane,
La figure 2 est une vue en perspective explosée du cotyle de la figure 1,
La figure 3 est une vue en coupe transversale verticale à travers le cotyle des figures 1 et 2, sur lequel on a mis en œuvre la présente invention, et
La figure 4 est une vue agrandie d'une partie de la figure 3, laissant plus clairement apparaitre la structure fine du cotyle de l'invention.

Un cotyle a été utilisé pour illustrer la présente invention, qui peut s'appliquer à toutes sortes d'implants destinés à venir en contact de l'os, sans utilisation de ciment ou autre liant.

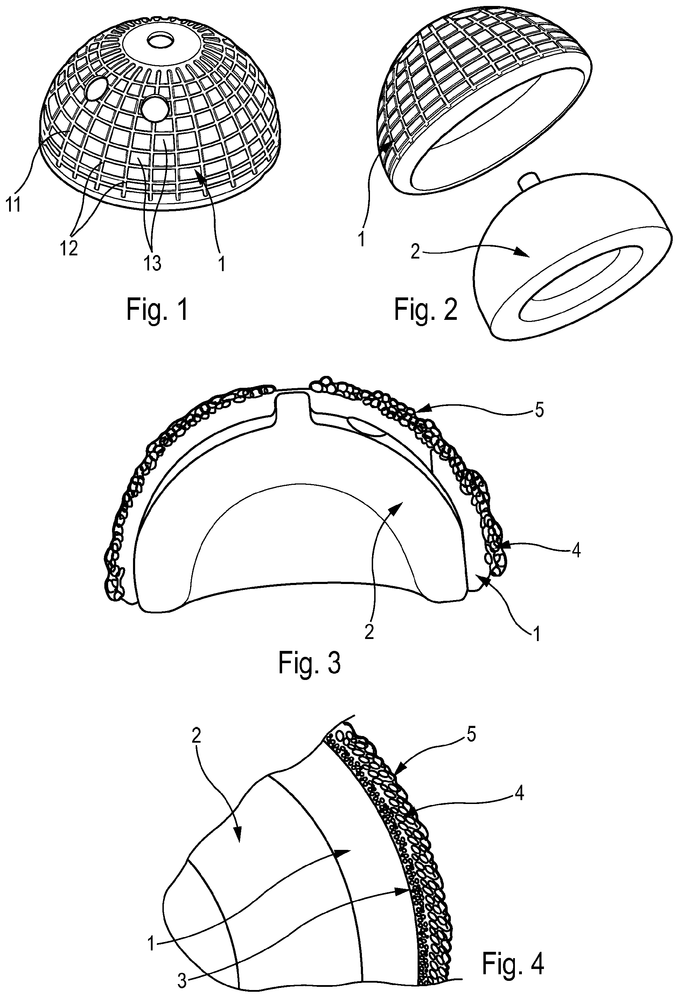

Le cotyle d'implant des figures 1 et 2 comprend un corps 1 qui est réalisé en titane ou en alliage de titane très dense : il n'est pas poreux. Ce corps 1 présente une forme de coque hémisphérique : son épaisseur peut être de l'ordre de 4 à 5 mm. Le corps est creux et contient un composant de frottement 2 qui peut être réalisé en Al₂O₃ ou en PE. Ce composant de frottement 2 forme donc une cavité interne, qui est destinée à recevoir la tête fémorale montée sur une tige fémorale. Extérieurement, le corps 1 définit une surface externe hémisphérique 11 qui peut être structurée, par exemple avec un réseau quadrillé de rainures 12, délimitant ainsi des petits pavés saillants 13. Cette macrostructure augmente la surface spécifique de contact, ce qui renforce la stabilité du cotyle dans la cavité osseuse de l'os iliaque. Il s'agit là d'une conception tout à fait classique pour un cotyle. De plus, la surface externe 11 du corps dense 1 présente de préférence une rugosité Ra de 4 µm à 8 µm.

Selon l'invention, la surface externe 11 du cotyle est recouverte par une d'une couche poreuse de titane ou d'alliage de titane, qui peut présenter une épaisseur de l'ordre de 200 µm à 400 µm, avantageusement de l'ordre de 300 µm à 350 µm. La rugosité du corps dense 1 permet une meilleure accroche de la couche poreuse.

Selon un mode de réalisation abouti, la couche poreuse peut comprendre une couche interne poreuse 3 en contact de la surface externe 11 et une couche externe poreuse 4 en contact de la couche interne poreuse 3. Les deux couches poreuses interne 3 et externe 4 présentent de préférence des porosités différentes : la couche externe poreuse 4 présentant avantageusement une porosité supérieure à celle de la couche interne poreuse 3. De préférence, la couche externe poreuse 4 peut présenter une porosité de l'ordre de 10 % à 25 %, avantageusement de l'ordre de 20 %. D'autre part, la couche externe poreuse 4 peut présenter une épaisseur de l'ordre de 150 µm à 300 µm. Par ailleurs, la couche externe poreuse 4 peut être constituée de poudre de titane ou d'alliage de titane ayant une granulométrie de l'ordre de 90 à 250 µm. La couche externe poreuse 4 présente de préférence une rugosité moyenne Ra de l'ordre de 30 à 70 µm et une rugosité maximale Rz de l'ordre de 150 à 300 µm. Enfin, la couche poreuse (interne et externe ensemble) peut présenter une adhérence supérieure ou égale à 22 MPa.

De son côté, la couche interne poreuse 3 peut présenter une épaisseur de l'ordre de 50 µm à 100 µm. Elle peut être constituée de poudre de titane ou d'alliage de titane ayant une granulométrie moyenne de l'ordre de 40 à 100 µm.

Des définitions classiques sont données au termes et expressions suivantes :
- Porosité : rapport du volume des vides au volume total,
- Granulométrie (moyenne) : taille (moyenne) des grains de poudre de titane,
- Rugosité moyenne Ra : écart moyen entre les pics et les creux, et
- Rugosité maximale Rz : rugosité maximale du profil.

Le dépôt de la couche externe 4 directement sur la surface externe 11 du cotyle n'est pas possible, ou du moins pas efficace, car l'adhérence des gros grains de poudre est difficile à réaliser. D'où la mise en œuvre de la couche interne avec des grains de poudre plus petits.

Selon l'invention, du PolyNaSS 5 est déposé par greffage, sur cette couche poreuse 4, étant donné qu'elle est en titane ou alliage de titane.

Un procédé de fabrication optimal comprend les étapes suivantes :
a- déposer à la torche à plasma de la poudre de titane ayant une granulométrie moyenne de l'ordre de 40 à 100 µm, de manière à former sur la surface externe 11 la couche interne poreuse 3 avec une épaisseur de l'ordre de 50 µm à 100 µm,
b- déposer à la torche à plasma de la poudre de titane ayant une granulométrie moyenne de l'ordre de 90 à 250 µm, de manière à former sur la couche interne poreuse 3 la couche externe poreuse 4 avec une épaisseur de l'ordre de 150 µm à 300 µm, et finalement
c- greffer du PolyNaSS 5 sur la couche externe poreuse 4.

La torche à plasma a permis d'obtenir une bonne adhérence de grains de poudre entre eux, tout en conservant une porosité optimale.

L'étape de greffage peut comprendre les étapes suivantes :
c1) Plonger les implants dans un bain d'acide pour les décaper,
c2) Rincer les implants,
c3) Plonger les implants dans un bain d'anodisation pour les anodiser,
c4) Rincer les implants,
c5) Introduire les implants dans une enceinte de polymérisation remplie de gaz inerte, tel que l'argon,
c6) Plonger les implants dans un bain de polymérisation présent dans l'enceinte,
c7) Soumettre le bain de polymérisation à un catalyseur de polymérisation pour synthétiser du polymère bioactif sur les implants,
c8) Extraire les implants de l'enceinte, et
c9) Laver les implants pour les débarrasser de l'excès de polymère bioactif non greffé.

Les chaines de PolyNaSS 5 peuvent ainsi s'accrocher à la couche poreuse qui est de surcroit particulièrement rugueuse et poreuse.

Le greffage du PolyNaSS sur une surface poreuse en titane procure un double avantage. Premièrement, le PolyNaSS est mieux accroché dans le relief perturbé de la couche externe 4. Deuxièmement, ce relief perturbé permet d'accrocher une plus grande quantité de PolyNaSS, ce qui favorise la repousse osseuse et l'anti-adhésion bactérienne.

Bien que l'invention ait été décrite en référence à un cotyle ayant un corps dense en titane ou en alliage de titane, elle s'applique à n'importe quel implant présentant un corps dense en titane ou en alliage de titane.

## Revendications

1. Implant comprenant un corps dense (1) en titane ou alliage de titane, revêtu d'une couche poreuse (3, 4) en titane ou alliage de titane, **caractérisé en ce que** la couche poreuse (3, 4) comprend un dépôt de PolyNaSS (5) appliqué par greffage.

2. Implant selon la revendication 1, dans lequel le corps dense (1) présente une rugosité Ra de 4 µm à 8 µm.

3. Implant selon l'une quelconque des revendications précédentes, dans lequel la couche poreuse (3, 4) présente une épaisseur de l'ordre de 200 µm à 400 µm, avantageusement de l'ordre de 300 µm à 400 µm.

4. Implant selon l'une quelconque des revendications précédentes, dans lequel la couche poreuse comprend une couche interne poreuse (3) en contact du corps dense (1) et une couche externe poreuse (4) en contact de la couche interne poreuse (3), les deux couches poreuses interne (3) et externe (4) présentant des porosités différentes, la couche externe poreuse (4) présentant avantageusement une porosité supérieure à celle de la couche interne poreuse (3).

5. Implant selon l'une quelconque des revendications précédentes, dans lequel la couche externe poreuse (4) présente une porosité de l'ordre de 10 % à 25 %, avantageusement de l'ordre de 20 %.

6. Implant selon l'une quelconque des revendications précédentes, dans lequel la couche externe poreuse (4) présente une épaisseur de l'ordre de 150 µm à 300 µm.

7. Implant selon l'une quelconque des revendications précédentes, dans lequel la couche externe poreuse (4) est constituée de poudre de titane ou d'alliage de titane ayant une granulométrie moyenne de l'ordre de 90 à 250 µm.

8. Implant selon l'une quelconque des revendications précédentes, dans lequel la couche externe poreuse (4) présente une rugosité moyenne Ra de l'ordre de 30 à 70 µm et une rugosité maximale Rz de l'ordre de 150 à 300 µm.

9. Implant selon l'une quelconque des revendications précédentes, dans lequel la couche interne poreuse (4) présente une adhérence supérieure ou égale à 22 MPa.

10. Implant selon l'une quelconque des revendications précédentes, dans lequel la couche interne poreuse (3) présente une épaisseur de l'ordre de 50 µm à 100 µm.

11. Implant selon l'une quelconque des revendications précédentes, dans lequel la couche interne poreuse (3) est constituée de poudre de titane ou d'alliage de titane ayant une granulométrie moyenne de l'ordre de 40 à 100 µm.

12. Procédé de fabrication d'un implant comprenant un corps dense (1) en titane ou alliage de titane, couche poreuse (3, 4) en titane ou alliage de titane étant déposée à la torche à plasma sur le corps dense (1) du PolyNaSS (5) étant ensuite déposé par greffage sur la couche poreuse (3, 4).

13. Procédé de fabrication selon la revendication 12, comprenant les étapes suivantes :
a- déposer à la torche à plasma de la poudre de titane ayant une granulométrie moyenne de l'ordre de 40 à 100 µm, de manière à former sur le corps dense (1) une couche interne poreuse (3) ayant une épaisseur de l'ordre de 50 µm à 100 µm,
b- déposer à la torche à plasma de la poudre de titane ayant une granulométrie moyenne de l'ordre de 90 à 250 µm, de manière à former sur la couche interne poreuse (3) une couche externe poreuse (4) ayant une épaisseur de l'ordre de 150 µm à 300 µm, et
c- greffer du PolyNaSS (5) sur la couche externe poreuse (4).

## Patentansprüche

1. Implantat mit einem dichten Körper (1) aus Titan oder Titanlegierung, der mit einer porösen Schicht (3, 4) aus Titan oder Titanlegierung beschichtet ist, **dadurch gekennzeichnet, dass** die poröse Schicht (3, 4) eine PolyNaSS-Ablagerung (5) enthält, die durch Aufpfropfen aufgebracht ist.

2. Implantat nach Anspruch 1,
wobei der dichte Körper (1) eine Rauigkeit Ra von 4 µm bis 8 µm aufweist.

3. Implantat nach einem der vorhergehenden Ansprüche,
wobei die poröse Schicht (3, 4) eine Dicke im Bereich von 200 µm bis 400 µm, vorteilhafterweise im Bereich von 300 µm bis 400 µm, aufweist.

4. Implantat nach einem der vorhergehenden Ansprüche,
wobei die poröse Schicht eine poröse Innenschicht (3) in Kontakt mit dem dichten Körper (1) und eine poröse Außenschicht (4) in Kontakt mit der porösen Innenschicht (3) enthält, wobei die beiden porösen Schichten, nämlich die Innenschicht (3) und die Außenschicht (4), unterschiedliche Porositäten aufweisen, wobei die poröse Außenschicht (4) vorteilhafterweise eine höhere Porosität als die poröse Innenschicht (3) aufweist.

5. Implantat nach einem der vorhergehenden Ansprüche,
wobei die poröse Außenschicht (4) eine Porosität im Bereich von 10 % bis 25 %, vorteilhafterweise im Bereich von 20 %, aufweist.

6. Implantat nach einem der vorhergehenden Ansprüche,
wobei die poröse Außenschicht (4) eine Dicke im Bereich von 150 µm bis 300 µm aufweist.

7. Implantat nach einem der vorhergehenden Ansprüche,
wobei die poröse Außenschicht (4) aus Titan- oder Titanlegierungspulver mit einer mittleren Korngröße im Bereich von 90 bis 250 µm besteht.

8. Implantat nach einem der vorhergehenden Ansprüche,
wobei die poröse Außenschicht (4) eine mittlere Rauigkeit Ra im Bereich von 30 bis 70 µm und eine maximale Rauigkeit Rz im Bereich von 150 bis 300 µm aufweist.

9. Implantat nach einem der vorhergehenden Ansprüche,
wobei die poröse Innenschicht (4) eine Haftfestigkeit von 22 MPa oder mehr aufweist.

10. Implantat nach einem der vorhergehenden Ansprüche,
wobei die poröse Innenschicht (3) eine Dicke im Bereich von 50 µm bis 100 µm aufweist.

11. Implantat nach einem der vorhergehenden Ansprüche,
wobei die poröse Innenschicht (3) aus Titan- oder Titanlegierungspulver mit einer mittleren Korngröße im Bereich von 40 bis 100 µm besteht.

12. Verfahren zur Herstellung eines Implantats mit einem dichten Körper (1) aus Titan oder Titanlegierung, wobei eine poröse Schicht (3, 4) aus Titan oder Titanlegierung mit einem Plasmabrenner auf den dichten Körper (1) aufgebracht wird und anschließend PolyNaSS (5) durch Aufpfropfen auf die poröse Schicht (3, 4) aufgebracht wird.

13. Herstellungsverfahren nach Anspruch 12, umfassend die folgenden Schritte:
a- Plasmabrennen von Titanpulver mit einer mittleren Korngröße im Bereich von 40 bis 100 µm, um auf dem dichten Körper (1) eine poröse Innenschicht (3) mit einer Dicke im Bereich von 50 µm bis 100 µm zu bilden,
b- Plasmabrennen von Titanpulver mit einer mittleren Korngröße im Bereich von 90 bis 250 µm, um auf der porösen Innenschicht (3) eine poröse Außenschicht (4) mit einer Dicke im Bereich von 150 µm bis 300 µm zu bilden, und
c- Aufpfropfen von PolyNaSS (5) auf die poröse Außenschicht (4).

## Claims

1. An implant comprising a dense body (1) made of titanium or titanium alloy, coated with a porous layer (3, 4) made of titanium or titanium alloy, **characterized in that** the porous layer (3, 4) comprises a deposition of PolyNaSS (5) applied by grafting.

2. The implant according to claim 1, wherein the dense body (1) has a roughness Ra of 4 µm to 8 µm.

3. The implant according to any one of the preceding claims, wherein the porous layer (3, 4) has a thickness of the order of 200 µm to 400 µm, advantageously of the order of 300 µm to 400 µm.

4. The implant according to any one of the preceding claims, wherein the porous layer comprises a porous inner layer (3) in contact with the dense body (1) and a porous outer layer (4) in contact with the porous inner layer (3), the two porous inner (3) and outer (4) layers having different porosities, the porous outer layer (4) advantageously having a porosity greater than that of the porous inner layer (3).

5. The implant according to any one of the preceding claims, wherein the porous outer layer (4) has a porosity of the order of 10% to 25%, advantageously of the order of 20%.

6. The implant according to any one of the preceding claims, wherein the porous outer layer (4) has a thickness of the order of 150 µm to 300 µm.

7. The implant according to any one of the preceding claims, wherein the porous outer layer (4) is made of titanium or titanium alloy powder having an average particle size of the order of 90 to 250 µm.

8. The implant according to any one of the preceding claims, wherein the porous outer layer (4) has an average roughness Ra of the order of 30 to 70 µm and a maximum roughness Rz of the order of 150 to 300 µm.

9. The implant according to any one of the preceding claims, wherein the porous inner layer (4) has an adhesion greater than or equal to 22 MPa.

10. The implant according to any one of the preceding claims, wherein the porous inner layer (3) has a thickness of the order of 50 µm to 100 µm.

11. The implant according to any one of the preceding claims, wherein the porous inner layer (3) is made of titanium or titanium alloy powder having an average particle size of the order of 40 to 100 µm.

12. A method for manufacturing an implant comprising a dense body (1) made of titanium or titanium alloy, porous layer (3, 4) made of titanium or titanium alloy being deposited using a plasma torch on the dense body (1) of the PolyNaSS (5) then being deposited by grafting on the porous layer (3, 4).

13. The manufacturing method according to claim 12, comprising the following steps:
a- depositing titanium powder having an average particle size of the order of 40 to 100 µm with a plasma torch, so as to form on the dense body (1) a porous inner layer (3) having a thickness of the order of 50 µm to 100 µm,
b- depositing titanium powder having an average particle size of the order of 90 to 250 µm with a plasma torch, so as to form on the porous inner layer (3) a porous outer layer (4) having a thickness of the order of 150 µm to 300 µm, and
c- grafting PolyNaSS (5) onto the porous outer layer (4).
